# EUROPEAN PATENT APPLICATION

(11) **EP 1 512 372 A1**
(43) Date of publication of application: **09.03.2005**
(21) Application number: 04104291.2
(22) Date of filing: 06.09.2004
(51) Int. Cl.: A61B 5/103, G06T 7/00, G06F 19/00

(54) **Method and device for quantidying the extension of a colour-altered skin or nail area**

(30) Priority: 05.09.2003 IT TO20030678
(71) Applicant: DERMING S.r.l., 20052 MONZA (IT)
(72) Inventor: Setaro, Michele, 20052, MONZA (IT); Sparavigna, Adele, 20052, MONZA (IT)
(74) Representative: Jorio, Paolo, Dr. Ing.

(57) **Abstract**

A method of quantifying the extension of a colour-altered skin or nail area, in particular a diseased nail area, includes the steps of: acquiring an image (1b) of the colour-altered area (3) and a normal adjacent area (4); selecting one of the areas (3, 4) as a work area (15); selecting a number of sample points (17) in the work area (15), and determining the colourimetric coordinates of the sample points (17); plotting the colourimetric coordinates of the sample points (17) in a colourimetric space, and defining, in the colourimetric space, a colour region (20) including the sample points (17) and representing all the points in the work area (15); calculating how many points in the image (1b) fall within the colour region (20) to obtain a value indicating the extension of the work area (15).

## Description

The present invention relates to a method and device for quantifying the extension of a colour-altered skin or nail area, in particular a diseased nail area.

The invention also relates to a method of evaluating the effectiveness of treatment and/or drugs and/or cosmetics on skin or nail areas, and in particular of the treatment of onychomycosis or other nail diseases.

Various skin and associated (nail, hair) diseases are characterized by areas differing in colour from the surrounding healthy areas. For example, a nail area affected by onychomycosis has a much different surface colour from the healthy area of the nail.

The problem therefore arises of quantifying the extension of the diseased (i.e. colour-altered) area to objectively evaluate development of the disease and, in particular, its response to treatment.

Another problem lies in the colour range of onychomycosis areas varying widely from one subject to another and even in the same subject. That is, the colour of various onychomycosis areas in different subjects, and even in the same subject, is not referable to one colour or to a range of adjacent colours in any one colourimetric space.

The same also applies to other diseases, even ailments and simply flaws, involving colour alterations of the nails or skin.

At present, there are no methods or devices for quantifying a diseased skin or nail area, i.e. objectively evaluating the effectiveness of treatment of such areas.

It is therefore an object of the present invention to provide an objective method of quantifying the extension of a colour-altered skin or nail area, and in particular of a diseased (e.g. onychomycosis) nail area. In particular, it is an object of the invention to provide a method which is fast, reliable, accurate, and cheap and easy to implement.

It is a further object of the invention to provide a device by which to implement such a method.

It is a further object of the invention to provide an objective method of evaluating the effectiveness of therapeutic and/or cosmetic treatment, i.e. drugs and/or cosmetics, on skin and nail areas, and in particular of evaluating treatment of onychomycosis and other nail diseases, and which at the same time is fast, reliable, accurate, and cheap and easy to implement.

According to the present invention, there is provided a method and device for quantifying the extension of a colour-altered skin or nail area, in particular a diseased nail area, as claimed in the accompanying Claims 1 and 5 respectively.

The method and device according to the invention provide for quantifying, and therefore objectively determining, the extension of the diseased or otherwise altered area quickly, easily, reliably, and accurately.

A reliable, accurate, objective assessment can therefore be made quickly and easily of developments of the treated area, to determine, for example, the effectiveness of treatment and/or drugs and cosmetics.

The invention therefore also relates to a method of evaluating the effectiveness of therapeutic and/or cosmetic treatment, i.e. drugs and/or cosmetics, on skin and nail areas, and in particular of evaluating the effectiveness of treatment of onychomycosis and other nail diseases, as claimed in the accompanying Claim 6.

A non-limiting embodiment of the present invention will be described by way of example with reference to the accompanying drawings, in which:
Figure 1 shows, schematically, an image of a human skin or nail area comprising a colour-altered area;
Figure 2 shows, schematically, a device in accordance with the present invention for processing the Figure 1 image;
Figure 3 shows, schematically, digital processing of the Figure 1 image in accordance with the method of the present invention;
Figure 4 shows, schematically, a colourimetric space employed in the method according to the invention.

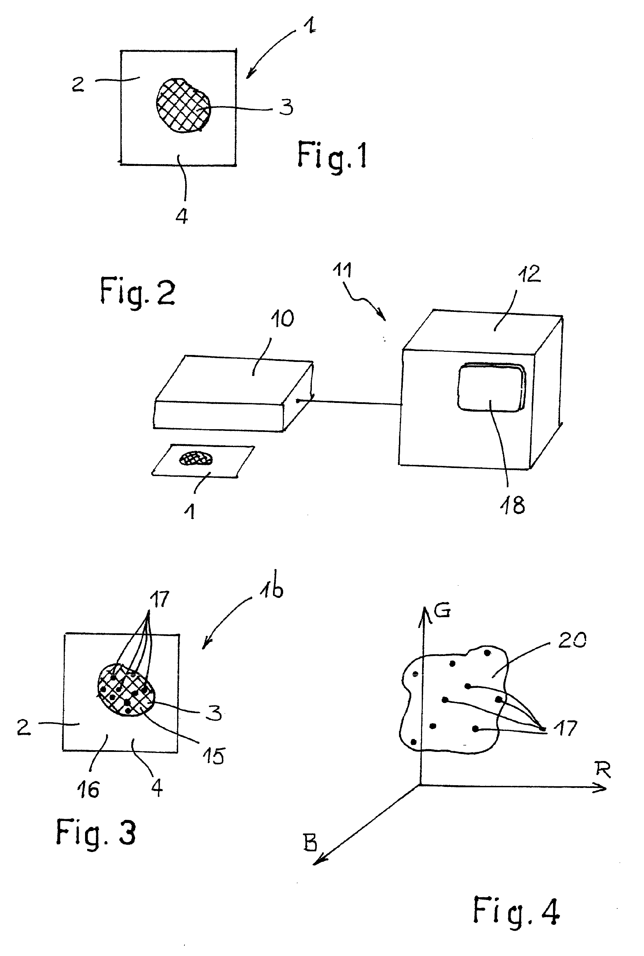

Figure 1 shows, schematically, an image 1 of a human skin or nail region 2 comprising a colour-altered diseased area 3, and a healthy area 4 of normal colour. For example, region 2 represents a nail (or a portion of it) affected by onychomycosis.

The method according to the invention provides for acquiring an image 1 of region 2 comprising the whole of altered area 3 and the whole nail, or at least a portion of the healthy nail adjacent to or surrounding altered area 3 and defining the normal area 4. Image 1 may be a photographic image, or any other type of image generated in any way.

With reference to Figure 2, image 1 is acquired in digital form, e.g. by means of a high-resolution scanner 10, by a device 11 comprising a processing unit 12 (e.g. a personal computer).

Processing unit 12 acquires a digital image 1b, corresponding to the original image 1 and shown schematically in Figure 3, and processes it as follows.

Area 3 or 4 is selected to define a work area 15, and the area 3 or 4 not selected defines a complementary area 16. Within work area 15 (which, in the example shown, is area 3, but which may equally be area 4), a number of sample points 17 are selected, each of which is basically a pixel of digital image 1b. Sample points 17 may be selected automatically by processing unit 12, e.g. on the basis of standard parameters memorized in processing unit 12, or may be operator-selected from image 1b displayed on a screen 18.

Each sample point 17 has a colour, which is acquired by processing unit 12 and expressed by colourimetric coordinates in any colourimetric space, e.g. an RGB space (red-green-blue space with the colours red, green, and blue along the cartesian axes.

As shown in Figure 4, the colourimetric coordinates of sample points 17 are plotted in the colourimetric space, in which they define a colour region 20 comprising all the sample points 17 and representing all the points in work area 15. All the points in work area 15 in fact have a colour included in colour region 20.

Though shown for the sake of simplicity in Figure 4 as a continuous volume of generic shape, colour region 20 may obviously comprise various, even separate, volumes of any shape.

At this point, processing unit 12 automatically calculates how many points or pixels in image 1b fall within colour region 20, i.e. how many points or pixels in image 1b are of a colour whose colourimetric coordinates in the colourimetric space fall within colour region 20.

Processing unit 12 also calculates automatically the total number of points or pixels in region 2 of image 1b.

More specifically, processing unit 12 acquires each point or pixel in image 1b, determines the colourimetric coordinates of each point, and determines whether the colourimetric coordinates fall within colour region 20 (in which case, the point defined by the colourimetric coordinates falls within work area 15) or are outside colour region 20 (in which case, the point defined by the colourimetric coordinates is outside work area 15 and falls within complementary area 16). Processing unit 12 thus counts, and so calculates the sum of, the points or pixels in work area 15, and the points or pixels in complementary area 16.

The number of points or pixels in colour region 20 is a value indicating the extension of work area 15, and which can be converted easily into a surface area measurement on the basis of a reference measurement acquired by processing unit 12.

Clearly, the method and device described herein with specific reference to onychomycosis may be used in all cases in which skin or associated areas, particularly nail and hair areas, are affected by colour alterations of any type or cause.

## Claims

1. A method of quantifying the extension of a colour-altered skin or nail area, in particular a diseased nail area, **characterized by** comprising the steps of:
- acquiring an image (1b) of the colour-altered area (3) and a normal adjacent area (4);
- selecting one of said areas (3, 4) as a work area (15) ;
- selecting a number of sample points (17) in the work area (15), and determining the colourimetric coordinates of said sample points (17);
- plotting the colourimetric coordinates of the sample points (17) in a colourimetric space, and defining, in said colourimetric space, a colour region (20) comprising the sample points (17) and representing all the points in the work area (15);
- calculating how many points in the image (1b) fall within the colour region (20) to obtain a value indicating the extension of the work area (15).

2. A method as claimed in Claim 1, **characterized in that** the image (1b) is acquired in digital form and processed by a processing unit (12).

3. A method as claimed in Claim 1 or 2, **characterized in that** the colour-altered area (3) and the normal area (4) respectively define the work area (15) and a complementary area (16) or vice versa.

4. A method as claimed in Claim 3, **characterized by** comprising the steps of acquiring each point in the image (1b); determining the colourimetric coordinates of each point; and determining whether said colourimetric coordinates fall within the colour region (20), in which case the point defined by said colourimetric coordinates is assigned to the work area (15), or fall outside the colour region (20), in which case the point defined by said colourimetric coordinates is assigned to the complementary area (16).

5. A device for quantifying the extension of a colour-altered skin or nail area, in particular a diseased nail area, **characterized by** comprising image-acquisition means (10) for acquiring an image (1b) of the colour-altered area (3) and a normal adjacent area (4); and processing means (12) for determining the colourimetric coordinates of a number of sample points (17) selected in one of said areas (3, 4) defined as a work area (15), defining in a colourimetric space a colour region (20) comprising the sample points (17), and calculating how many points in the image (1b) fall within the colour region (20) to obtain a value indicating the extension of the work area (15).

6. A method of evaluating the effectiveness of treatment and/or drugs and/or cosmetics on skin or nail areas, and in particular of the treatment of onychomycosis or other nail diseases, **characterized by** comprising the method as claimed in one of Claims 1 to 4.
